# EUROPÄISCHE PATENTANMELDUNG

(11) **EP 1 214 941 A1**
(43) Veröffentlichungstag der Anmeldung: **19.06.2002**
(21) Anmeldenummer: 00811159.3
(22) Anmeldetag: 07.12.2000
(51) Int. Cl.: A61K 33/08, A61K 33/14, A61K 33/42

(54) **Zubereitung zur Bahandlung von flächigen, offenen Wunden**

(71) Anmelder: Gehl, Gerolf, Dr. med. dent., 8700 Küsnacht (CH)
(72) Erfinder: Gehl, Gerolf, Dr. med. dent., 8700 Küsnacht (CH)
(74) Vertreter: Patentanwälte Schaad, Balass, Menzl & Partner AG

(57) **Zusammenfassung**

Zubereitung zur Behandlung von flächigen, offenen Wunden, die eine feste aktive Komponente aufweist, die ein Salz eines biologisch abbaubaren Phosphates sowie Calzium- und Alkalimetallionen enthält.

## Beschreibung

Die vorliegende Erfindung betrifft eine Zubereitung zur Behandlung von flächigen, offenen Wunden.

Wunden sind Schäden an Oberflächengeweben, die beispielsweise durch einen Dekubitus, Verbrennungen, die durch Wärme, UV-Strahlen oder Chemikalien hervorgerufen sind, operative Entfernungen, ausgefranste Wunden, Amputationen, Abschürfungen und Infektionen verursacht werden. Häufig ist die physische Verfassung der Patienten ebenfalls herabgesetzt. Es ist deshalb erwünscht, die Heilung positiv und direkt zu beschleunigen, ohne sich nur auf die natürliche Heilung von Wunden zu verlassen. Ferner ist die Pflege der Wunden sehr zeit- und kostenintensiv. Bei Dekubitus-Patienten leidet nicht nur der Patient selbst unter Schmerzen, sondern es fallen auch grosse Kosten zur Heilung solcher Wunden an.

Klassische Behandlungsmethoden umfassen unter anderem das Anbringen eines Hydrocolloidverbandes sowie feuchtes Verbinden mit Ringer-Lösung oder Lavasept® und einem okklusiven Verband.

Bei der konventionellen chirurgischen Defektdeckung werden in der Regel freie Lappentransplantate oder Schwenklappen verwendet. Durch Gefässanschlüsse kann dadurch versucht werden, die Durchblutung und Einheilung des Transplantates sicherzustellen. Allerdings führt die Entnahme der Transplantate zu neuen Wunden und Narben, was verständlicherweise nicht erwünscht ist. Zusätzlich ist diese mikrochirurgische Operationstechnik aufwendig und kostenintensiv.

Alternativ kann solchen Patienten durch den Einsatz von künstlichem Gewebe wie z.B. Apligraf® (Novartis Pharmaceutical Corporation) geholfen werden. Die Herstellung von künstlichem Gewebe ist jedoch sowohl zeitals auch kostenintensiv.

In DE-C 41 13 021 ist ein Implantat als Ersatz von Knochen beschrieben. Das Implantat besteht aus einem porösen, der Struktur des Knochens angepassten Material. Das Material wird im Laufe der Zeit vollständig resorbiert und durch Knochensubstanz ersetzt.

Aus DE-A 198 36 985 sind Implantate, insbesondere Brustimplantate, bekannt, die aus einem körperverträglichen, vom Körper resorbierbaren, keramischen Material mit einer Porenstruktur bestehen.

In EP-B 0 543 548 ist ein Implantat beschrieben, bestehend aus einem Granulat eines glasigen oder glasigkristallinen Materials, das zur Gewebsproliferation beiträgt.

Aufgabe der Erfindung ist es, eine Zubereitung zur Behandlung von flächigen, offenen Wunden anzugeben, die die Wundheilung deutlich beschleunigt.

Eine weitere Aufgabe der vorliegenden Erfindung ist es, eine Zubereitung zur Behandlung von flächigen, offenen Wunden anzugeben, die eine kostengünstige Alternative zu den bisher bekannten Behandlungsmöglichkeiten darstellt.

Eine weitere Aufgabe der vorliegenden Erfindung ist es, eine Zubereitung vorzuschlagen, die neben der wundheilenden Wirkung gleichzeitig als mikrobiozides Mittel bei infizierten Wunden wirkt.

Eine weitere Aufgabe der vorliegenden Erfindung ist es, eine Zubereitung zur Behandlung von nicht oder schlechtheilende Wunden von Diabetes- oder Dekubituspatienten vorzuschlagen.

Die Aufgabe wird gelöst durch eine Zubereitung zur Behandlung von flächigen, offenen Wunden dadurch gekennzeichnet, dass sie eine feste aktive Komponente aufweist, die ein Salz eines biologisch abbaubaren Phosphates sowie Calzium- und Alkalimetallionen enthält.

Besonders bevorzugt ist das Salz eines biologisch abbaubaren Phosphates ein Salz ausgewählt aus der Gruppe der Phosphorsäure, Metaphosphorsäure, Diphosphorsäure und die Gegenionen dieser Salze vorzugsweise Erdalkalimetallionen sind. Besonders bevorzugt sind Kalziummetaphosphat (Ca(PO₃)₂), β-Tricalziumphosphat Ca₃(PO₄)₂ und Hydroxylapathit Ca₃(PO₄)₃OH. Diese Salze eines biologisch abbaubaren Phosphates können jeweils alleine oder in Mischungen vorkommen.

In einer weiteren bevorzugten Ausführungsform sind die Alkalimetallionen Natrium- und/oder Kaliumionen. Diese können jeder Form von den entsprechenden Halogeniden oder Oxiden zu der Zubereitung zugegeben werden, oder sie können auch schon als Gegenionen der biologisch abbaubaren Phosphate neben den Erdalkalimetallionen vorkommen. Besonders bevorzugt sind Natrium-Calzium-Diphosphate und Kalium-Calzium-Diphosphate.

In einer weiteren bevorzugten Ausführungsform kommt neben Kalzium ein weiteres Erdalkalimetallion, nämlich Magnesium, vor. Dies kann als Gegenion der biologisch abbaubaren Phosphate, als Halogenidsalz oder als Oxid der Zubereitung zugegeben werden.

Als besonders geeignet haben sich Zubereitungen, die folgende Zusammensetzung aufweisen, erwiesen:

| | |
|---|---|
| P₂O₅ | 27.1 bis 39.9 mol% |
| CaO | 22.5 bis 36.0 mol% |
| M₂O | 14.5 bis 30.0 mol% |
| MgO | 5.0 bis 26 mol% |

wobei M₂O 27.1 bis 30.0 mol% Na₂O und 0 bis 12 mol% an K₂O enthält.

Zur Herstellung der erfindungsgemässen Zubereitung können die Ausgangsmaterialien bei 1150°C-1550°C geschmolzen werden. Das erhaltene glasartige Material wird daraufhin zerkleinert und gemahlen, so dass die erfindungsgemässe Zubereitung mit einer Partikelgrösse von 50 bis 250 µm, entsteht. Eine Partikelgrösse von 50 bis 150 µm ist besonders bevorzugt.

Die erfindungsgemässe Zubereitung wird direkt auf die Wunde aufgebracht oder aber auf einem - vorzugsweise festen - Träger appliziert. Kraterförmige Vertiefungen der Wunde werden mit der erfindungsgemässen Zubereitung ausgefüllt und eingeebnet. Anschliessend wird die Wunde ausreichend mit Flüssigkeit versorgt. Diese Flüssigkeit kann eine isotonische Lösung (wie zum Beispiel eine Ringer-Lösung) oder eine Lösung sein, die eine desinfizierende Wirkung aufweist (z.B. Lavasept® ). Bei der Applikation der erfindungsgemässen Zubereitung ist die Versorgung der Wunde mit Flüssigkeit von erheblicher Bedeutung. Wird zu wenig Flüssigkeit zugegeben, führt dies zu einer unerwünschten Verklumpung der erfindungsgemässen Zubereitung und zu einer Verkrustung am Wundrand. Die optimale Dosierung ist von der Wundgrösse und -tiefe abhängig und vom Fachmann bestimmt werden. In einer bevorzugten Ausführungsform wird die Flüssigkeitszufuhr durch Drainagen gewährleistet. Schliesslich wird die Wunde grossflächig abgedeckt. Dies kann durch den Träger selbst oder durch verschiedenste Verbandmaterialien wie Hydrocolloidfolien, vollbracht werden.

In einer bevorzugten Ausführungsform enthält der Träger einen Material, das sich bei Temperaturen oberhalb der Körpertemperatur verformen lässt, wie zum Beispiel Wachs. Direkt vor der Verwendung kann ein solcher Träger erweicht werden. Dieser weiche Träger kann nun der Form der Wunde bzw. des zu behandelnden Körperteils angepasst werden. Durch dieses Vorgehen können weitere Druckstellen vermieden werden. Weiterhin ist es möglich, die Wunde so optimal abzuschliessen.

Die erfindungsgemässe Zubereitung stellt vermutlich dem Körper, die für die Gewebsbildung wichtigen Ionen poolartig zur Verfügung. Die Wirkung der erfindungsgemässen Zubereitung beruht wahrscheinlich auf dem Ionenaustausch der molekularen Bestandteile in Wechselwirkung mit dem Gewebe. Die Ionenabgabe wirkt dabei vermutlich bioinduktiv, d.h. die Gewebsproliferation wird angeregt. Mithin fördert das Pulver die Wundheilung im Sinne einer Proliferation des Bindegewebes mit Kapillargefässbildung. So wie sich dies zeigt, wird eine grossflächige Wunde mit frischem Gewebe aufgefüllt. Durch die erfindungsgemässe Zubereitung findet eine Volumenauffüllung der offenen Wunde statt, die beim Gefässanschluss notwendig ist und mithin muss kein gut durchblutetes Transplantat mehr am Wunddefekt adaptiert werden. Vielmehr reicht es aus, dass die erfindungsgemässe Zubereitung in die angefrischte, leicht blutende Wunde angebracht wird. Offenbar wird eine Proliferation und Einsprossung des Gewebes durch erfindungsgemässe Zubereitung provoziert. Während das Weichgewebe aus der Tiefe und vom Epithelrand vordringt, wird es gleichzeitig von neu gebildeten Kapillaren durchzogen, durchblutet und ernährt, was darin resultiert, dass kein chirurgischer Eingriff mehr notwendig ist. Auf diese Weise wird eine tiefe und grossflächige Wunde mit frischem Gewebe ausgefüllt. Eine beschichtete Hydrokoloidfolie dient für das einwachsende Gewebe als limitierende Grenzfläche, da diese Folie eine Epithelisierung induziert und deshalb nach erfolgten Volumenausgleich zur Abheilung der Wunde mittels Epithelbildung führt.

In einem der nachfolgenden Beispiele, zeigt die erfindungsgemässe Zubereitung ausserdem eine bakterizide Wirkung.

Durch die erfindungsgemässe Zubereitung wird auf jede chirurgische Massnahme verzichtet. Die Wunde wird weder vernäht noch verschlossen, sondern es handelt sich um eine offene Wundheilung. Deshalb kann dieses Verfahren von jedem Hausarzt ambulant durchgeführt werden, was zu einer erheblichen Kostendämpfung in der Behandlung schlecht heilender Wunden führt.

Die erfindungsgemässe Zubereitung kann alleine oder in einer Verpackungseinheit mit einer separat verpackten isotonischen Lösung oder einer separat verpackten desinfizierend wirkenden Lösung an den Kunden abgegeben werden, um so den individuellen Bedürfnissen gerecht zu werden.

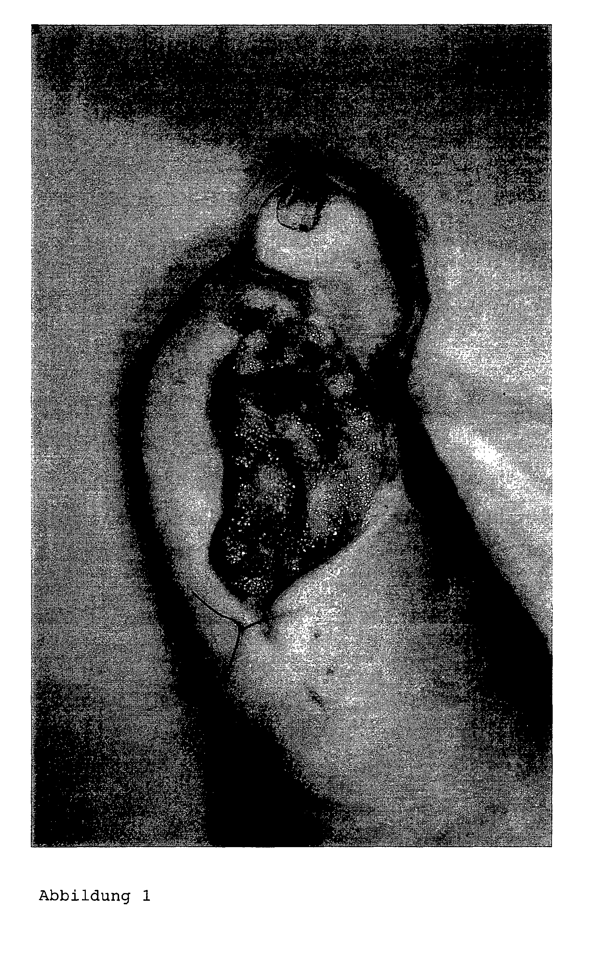

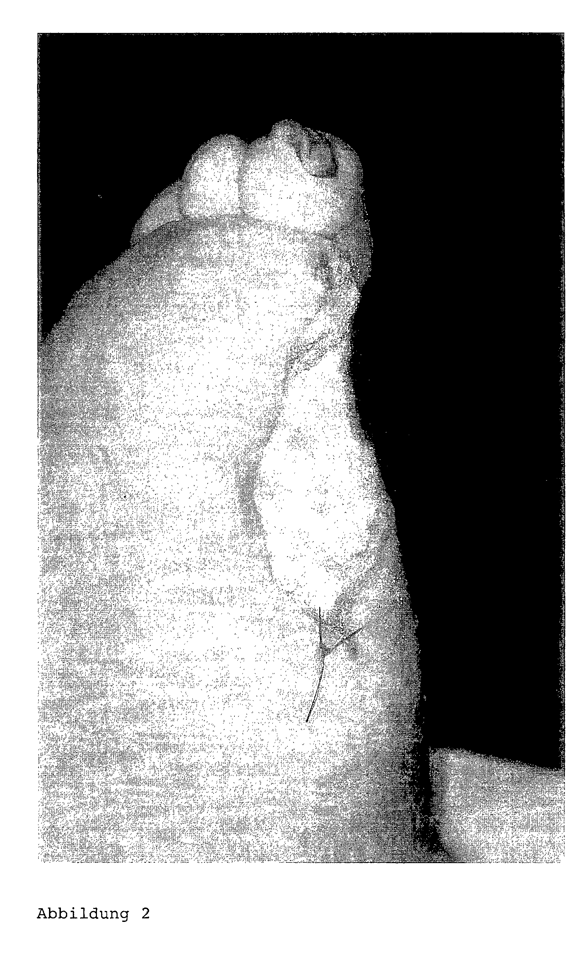

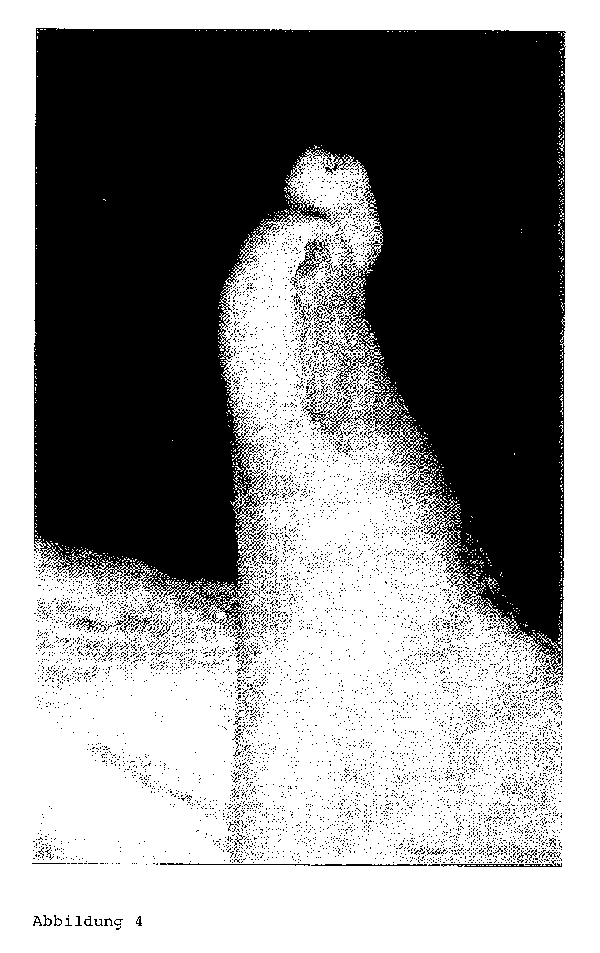

Die Abbildungen 1 bis 4 zeigen den Wundheilungsprozess einer offenen flächigen Wunde bei der Behandlung mit der erfindungsgemässen Zubereitung im Verlauf der Zeit (vergleiche auch Beispiel 13).

Abbildung 1 zeigt die unbehandelte offene flächige Wunde.

Abbildung 2 zeigt die mit der erfindungsgemässen Zubereitung flächendeckend bestreuten Wunde.

Abbildung 3 zeigt die mit der erfindungsgemässen Zubereitung behandelte Wunde nach 10 Tagen. Die offene Wunde ist schon deutlich kleiner geworden.

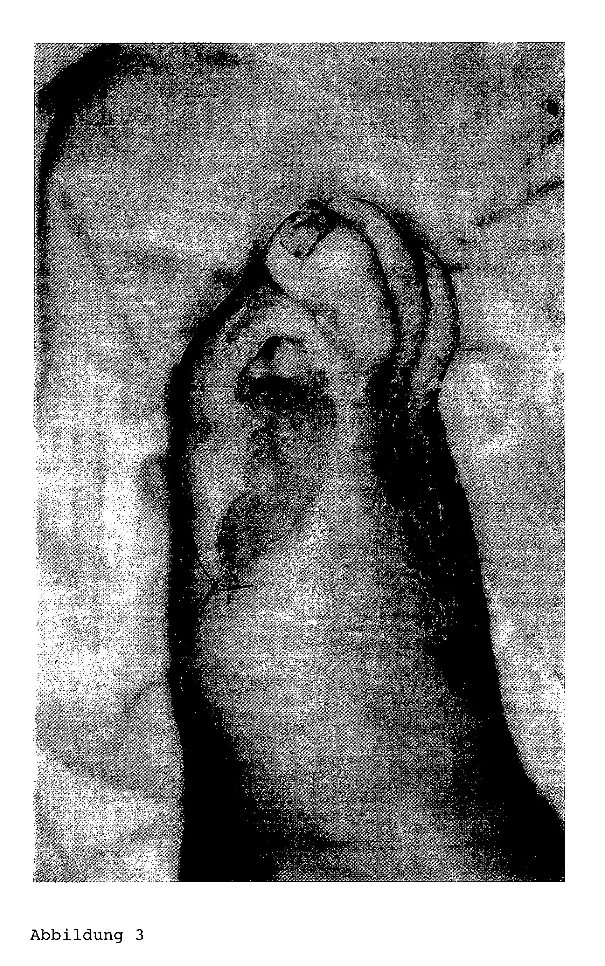

Abbildung 3 zeigt die mit der erfindungsgemässen Zubereitung behandelte Wunde nach 24 Tagen. Ein weiterer Heilungsfortschritt ist zu erkennen.

### Beispiel 1

Glaskeramik der Zusammensetzung

| | |
|---|---|
| P₂O₅ | 32.6 mol% |
| CaO | 27.6 mol% |
| Na₂O | 27.6 mol% |
| MgO | 12.2 mol% |

wird bis zum Erhalt der gewünschten Korngrösse gemahlen.

### Beispiele 2-12

Analoge Herstellung der erfindungsgemässen Zubereitung, die folgende Zusammensetzung (in mol%) aufweist:

| | **2** | **3** | **4** | **5** | **6** | **7** | **8** | **9** | **10** | **11** | **12** |
|---|---|---|---|---|---|---|---|---|---|---|---|
| **P**_{**2**}**O**_{**5**} | 32.6 | 32.6 | 32.6 | 32.6 | 37.0 | 27.1 | 35.5 | 38.9 | 36.3 | 34.0 | 29.8 |
| **CaO** | 27.6 | 27.6 | 27.6 | 27.6 | 29.1 | 24.5 | 27.8 | 30.6 | 27.6 | 28.1 | 26.1 |
| **Na**_{**2**}**O** | | 13.8 | 6.9 | 20.7 | 11.9 | 23.4 | 11.4 | 25.5 | 29.6 | 26.5 | 28.4 |
| **K**_{**2**}**O** | 27.6 | 13.8 | 20.7 | 6.9 | 12.0 | | 11.4 | | | | |
| **MgO** | 12.2 | 12.2 | 12.2 | 12.2 | 10.0 | 25 | 13.9 | 5 | 6.4 | 11.4 | 15.7 |

### Beispiel 13

### Pilotversuch an einem Einzelpatienten

### Krankengeschichte:

Seit ca. 1.5j besteht rechts ein Malum perforans. Operation wegen Osteomyelitis im August 2000 in Bülach mit Teilamputation des rechten Metatarsale I und II.

Zwischenzeitlich Pflege zu Hause durch die Spitex.

Am 23.9.00 entwickelte sich im Bereich der rechten Ferse ein nekrotisches Areal. In der Folge dann Fieber und beginnende perifokale Rötung. Der Hausarzt begann eine antibiotische Th. mit Augmentin 2x1g p.o.

**Tag 1:** bis anhin komplikationsloser postoperativer Verlauf, regelmässiger Verbandswechsel 2x/d mit feuchten Verbänden

**Tag 4:** Wundkontrolle: Die Wunde anterior bei der Operationsstelle misst ca. 8cmx5cm, granuliert gut, keine Zeichen von Infektion. Die Ferse ist wesentlich stark belegt mit Fibrin.

**Tag 7:** Operationsstelle sieht gut granulierend aus, anterior jedoch ein leiht nekrotisches Täschlein. Debridement durchgeführt, weiter mit feuchtem Verbinden. Ferse debridiert: ab nächste Woche Versuch mit erfindungsgemässer Zubereitung. Weiter mit TEAM-system.

**Tag 10:** Wundkontrolle: Debridement anterior & posterior. Erfindungsgemässe Zubereitung wurde auf die Wunde gestreut. Verbinding anterior & posterior feucht. Wundkontrolle jede 2 Tage.

**Tag 13:** Die Wunde an der Ferse stark übelriechend, grünlich belegte Kompressen. Vd.a. Pseudomonas, Wundabstrich genommen. Weiterhin mit nicht-okklusiven Verbänden.

**Tag 14:** Wundkontrolle: Wunden stinken weniger, die Operationswunde sieht sehr gut aus, jedoch gibt es ein Täschlein anterior mit Verdacht dass die Knochen des Dig III freigelegt sind. Wunde an der Ferse sieht peripher gut aus, zentral stets nekrotisch. Debridiert, erfindungsgemässe Zubereitung erneut auf beiden Wunden, mit Lavasept verbunden (nicht okklusiv).

**Tag 17:** Wundkontrolle: Die Wunde posterior ist auf dem Calcanues. Beide Wunden weisen jedoch Zeichen von Granulation. Weiter mit der erfindungsgemässen Zubereitung.

**Tag 18:** Wundkontrolle: Anterior wieder schön, beginnende Epithelialisation von peripher. Wunde an der Ferse stets zentral trocken mit Calcaneus, peripher granulierend.

**Tag 20:** Wundkontrolle: die Wunden sehen beide sehr gut aus. Umstieg von feuchtem, nicht-okklusivem Verbinden auf Replicare (semi-okklusiv).

Wundabstrich-Resultate:
- Grammnegative Stäbchen: reichlich vorhanden
- E. coli: reichlich vorhanden
- Enterokokken: reichlich vorhanden
- Keime der Hautflora: reichlich vorhanden

**Tag 24:** Rückfrage wegen starkem Juckreiz (Ekzem). Therapievorschlag: Diprogenta am Unterschenkel bds, 1% Hydrocortison in ColdCreme am übrigen Körper. Prednison 100mg tgl, ausschleichend über 10 Tage.

Wundkontrolle: sehr gute Wundverhältnisse, ausser im Täschlein anterior, und im Mittelpunkt der Ferse. vorgesehen ist eine Operation mit Öffnung der Tasche und allenfalls Amputation Dig III, sowie Corticalis-Entfernung an der Ferse. Später am Tag lehnte der Patient die Operation ab.

**Tag 25:** Juckreiz heute stark regredient.

**Tag 26:** Wundkontrolle: Anterior ist die Wunde deutlich kleiner, das Täschlein ganz vorne jedoch stets schlecht granulierend. Posterior auch gute Granulationsgewebe mit Epithelialisation von der Peripherie, jedoch zentral noch zum Knochen. Zentral debridiert.

**Tag 28:** Wundkontrolle: immerhin gute Wundverhältnisse ausser ganz anterior (Täschlein), sowie Mitte der Wunde an der Ferse. Leicht erhöhte Blutzucker-Werte bei Prednison-Einnahme.

**Tag 30:** Verbandwechsel: hypertrophes Granulationsgewebe an der Ferse.

**Tag 32:** Verbandwechsel, zum letzten Mal mit erfindungsgemässer Zubereitung verbunden, und mit Replicare abgedeckt. Ferse zentral debridiert. Granulationsgewebe gut durchblutet. Wunde anterior kleiner, Täschlein sauber aber stets ca. 2cm tief.

**Tag 34:** gut granulierende Wundverhältnisse, Zentral eher besser (Knochen scheint weniger freigelegt zu sein). Wunde anterior epithelialisierend, Täschlein 2cm, serös. Verbindung anterior mit VacVerband (Coloplast herum zur Hautschutz). Posterior nach TEAM-System (Allevyn & Opsite).

## Patentansprüche

1. Zubereitung zur Behandlung von flächigen, offenen Wunden **dadurch gekennzeichnet, dass** sie eine feste aktive Komponente aufweist, die ein Salz eines biologisch abbaubaren Phosphates sowie Calzium- und Alkalimetallionen enthält.

2. Zubereitung gemäss Anspruch 1 **dadurch gekennzeichnet, dass** das Salz eines biologisch abbaubaren Phosphates ein Salz ist ausgewählt aus der Gruppe der Phosphorsäure, Metaphosphorsäure sowie Diphosphorsäure und Gegenionen vorzugsweise Erdalkalimetallionen enthalten.

3. Zubereitung gemäss einem der Ansprüche 1 und 2 **dadurch gekennzeichnet, dass** das Salz eines biologisch abbaubaren Phosphates ein Salz ist ausgewählt aus der Gruppe von Calziummetaphosphat (Ca(PO₃)₂), β-Tricalziumphosphat (Ca₃(PO₄)₂) sowie Hydroxylapathit (Ca₃(PO₄)₃OH).

4. Zubereitung gemäss einem der vorangehenden Ansprüche **dadurch gekennzeichnet, dass** die Alkalimetallionen Natrium- und/oder Kaliumionien sind.

5. Zubereitung gemäss einem der vorangehenden Ansprüche **dadurch gekennzeichnet, dass** sie zusätzlich Magnesiumionen enthält.

6. Zubereitung gemäss einem der vorangehenden Ansprüche **dadurch gekennzeichnet, dass** sie 27.1 bis 39.9 mol% P₂O₅, 22.5 bis 36.0 mol% CaO, 14.5 bis 30.0 mol% M₂O und 5.0 bis 25 mol% MgO enthält,
wobei M₂O 27.1 bis 30.0 mol% Na₂O und 0 bis 12 mol% an K₂O enthält.

7. Zubereitung gemäss einem der vorangehenden Ansprüche **dadurch gekennzeichnet, dass** sie in Form eines Pulvers vorliegt.

8. Zubereitung gemäss Anspruch 7 **dadurch gekennzeichnet, dass** das Pulver eine Partikelgrösse von 50 bis 250 µm aufweist.

9. Zubereitung gemäss einem der vorangehenden Ansprüche, **dadurch gekennzeichnet, dass** sie auf einen Träger appliziert ist.

10. Zubereitung gemäss einem der vorangehenden Ansprüche **dadurch gekennzeichnet, dass** der Träger bei Temperaturen über Körpertemperatur verformbar ist.

11. Zubereitung gemäss Anspruch 10 **dadurch gekennzeichnet, dass** der Träger Wachs enthält.

12. Verpackungseinheit enthaltend eine Zubereitung nach einem der Ansprüche 1 bis 11 und eine isotonischen Lösung.

13. Verpackungseinheit enthaltend eine Zubereitung nach einem der Ansprüche 1 bis 11 und eine desinfizierend wirkende Lösung.

14. Kombination eines biologisch abbaubaren festen Phosphates, Calzium- und Alkalimetallionen als Mittel zur Behandlung von flächigen, offenen Wunden.
